# EUROPEAN PATENT APPLICATION

(11) **EP 4 715 015 A1**
(43) Date of publication of application: **25.03.2026**
(21) Application number: 24807205.0
(22) Date of filing: 14.05.2024
(51) Int. Cl.: C09C 3/06, A61K 8/19, A61K 8/25, A61K 8/26, A61K 8/27, A61K 8/28, A61K 8/29, A61Q 1/04, A61Q 1/10, C09C 3/08

(54) **BRILLIANT PIGMENT, PIGMENT-CONTAINING COMPOSITION, AND PIGMENT-CONTAINING COATED BODY**

(30) Priority: 15.05.2023 JP 2023080336
(71) Applicant: Nippon Sheet Glass Company, Limited, Tokyo 108-6321 (JP)
(72) Inventor: HORIGUCHI, Haruko, Tokyo 108-6321 (JP)
(74) Representative: Cabinet Beau de Loménie
(86) International application number: PCT/JP2024/017832
(87) International publication number: WO 2024/237253

(57) **Abstract**

Provided is a glitter pigment including a flaky substrate, an optical interference film on the flaky substrate, and a composite particle including a pigment particle and an oxide particle, the composite particle being attached to the optical interference film. The pigment particle includes, for example, an organic pigment. The oxide particle includes, for example, at least one selected from the group consisting of silicon oxide, aluminum oxide, zirconium oxide, titanium oxide, zinc oxide, iron oxide, tin oxide, niobium oxide, and cerium oxide. The optical interference film includes, for example, titanium oxide.

## Description

### TECHNICAL FIELD

The present invention relates to a glitter pigment, more specifically, a glitter pigment including a flaky substrate and an optical interference film on the flaky substrate. Furthermore, the present invention relates to a pigment-containing composition and a pigment-containing painted article, both of which contain the glitter pigment.

### BACKGROUND ART

A glitter pigment exhibits a pearlescent luster due to optical interference effect of an optical interference film. Such a glitter pigment is added to products such as paints and cosmetics, thereby imparting a grainy and vivid light reflection to the products. A representative example of the optical interference film is a titanium oxide film. A glitter pigment that uses a colorant together with an optical interference film is also known. For example, Patent Literature 1 discloses a cosmetic additive including mica coated with a titanium oxide film (titanium oxide-coated mica), the mica being further coated with a red organic colorant.

### CITATION LIST

### Patent Literature

Patent Literature 1: JP 2004-315426 A

### SUMMARY OF INVENTION

### Technical Problem

Employment of a colorant is useful as a method for adjusting color tones or the like of reflected light from a glitter pigment. However, according to the investigations by the present inventor, desired reflected light may not be obtained from a glitter pigment prepared by attaching a pigment particle as a colorant onto an optical interference film. The present invention aims to provide a novel glitter pigment that includes a pigment particle together with an optical interference film.

### Solution to Problem

The present invention provides a glitter pigment including:
a flaky substrate;
an optical interference film on the flaky substrate; and
a composite particle including a pigment particle and an oxide particle, the composite particle being attached to the optical interference film.

### Advantageous Effects of Invention

According to the present invention, a novel glitter pigment including a pigment particle together with an optical interference film is provided.

The following describes the details of the present invention, though the following description is not intended to limit the present invention to specific embodiments. In the present Description, "main component" refers to a component having the highest content in terms of mass. The average particle diameter can be determined based on the observation results regarding 20 or more objects, or preferably 50 objects, the results being obtained by using a scanning electron microscope (SEM) or the like. The average particle diameter is, for example, the arithmetic mean of the particle diameters of individual objects, the particle diameters are determined by averaging the longest diameter and the diameter in a direction perpendicular to the longest diameter at the midpoint of the longest diameter.

### (Flaky substrate)

A flaky substrate is a small and plate-like thin piece, which is also known as a scale-shaped substrate. Examples of the flaky substrate include a glass flake, an alumina flake, mica, talc, and sericite. The flaky substrate is preferably a glass flake, an alumina flake, or mica. The mica may be either natural mica or synthetic mica.

A preferred average thickness of the flaky substrate is 5 µm or less, particularly 2 µm or less, for example, 0.1 to 5 µm, or even 0.15 to 2 µm. The average value of thickness of the flaky substrate is determined by the average thickness of 20 or more flaky substrates, preferably 50 flaky substrates. The thickness of each flaky substrate can be measured by observation using a scanning electron microscope (SEM). The preferred average particle diameter of the flaky substrate is 3 to 600 µm, particularly 4 to 500 µm, or for example, 5 to 200 µm.

The most preferred flaky substrate is a glass flake. The main surface of the glass flake is smoother in comparison with a surface of a crystalline particle such as mica, and thus, the glass flake is suitable for exhibiting vivid interference light due to the interference of incident light. The glass composition constituting the glass flake is not particularly limited, but examples of applicable glass compositions include silicon oxide as the main component and further include components of metal oxide such as aluminum oxide, calcium oxide, sodium oxide or the like. Specific examples of the glass compositions include soda-lime glass, A-glass, C-glass, E-glass, ECR glass, borosilicate glass, and aluminosilicate glass.

### (Optical interference film)

An optical interference film may include at least one component selected, for example, from the group consisting of silicon oxide, aluminum oxide, zirconium oxide, titanium oxide, zinc oxide, iron oxide, tin oxide, niobium oxide, cerium oxide, nickel oxide, chromium oxide, and vanadium oxide. The optical interference film may be a single layer or may be formed of a plurality of layers. Each layer constituting the optical interference film may include, for example, at least one of the aforementioned oxides, and preferably includes any of the aforementioned oxides as its main component. The optical interference film may include titanium oxide, and may include a layer containing titanium oxide as the main component.

The optical interference film exhibits diverse interference light depending on the number and arrangement of the layers and depending on the materials constituting the respective layers. For example, a titanium oxide film as a single layer imparts interference colors such as yellow at approximately 100 nm thickness, red at approximately 130 nm thickness, blue at approximately 160 nm thickness, and green at approximately 175 nm thickness. However, even with the same film thickness, the color tone of the titanium oxide may vary slightly depending on film formation conditions or any other factors.

Titanium oxide has a high refractive index, and thus, it is suitable for forming a layer with excellent color expression properties. Titanium oxide can have three types of crystal structures, namely, anatase-type, brookite-type, and rutile-type structures, and anatase-type titanium oxide and rutile-type titanium oxide are industrially manufactured. The rutile-type structure is preferred among the crystal structures of titanium oxide. Rutile-type titanium oxide has a low photocatalytic activity and thus has a low impact on a matrix material, such as a paint, to which the glitter pigment is added. And the rutile-type titanium oxide has the highest refractive index.

Formation of a rutile-type titanium oxide film on the flaky substrate may be carried out, for example, according to a method disclosed in JP 2001-031421 A, JP 2003-012962 A or the like. In the method disclosed in these patent publications, rutile-type titanium oxide is precipitated on glass flakes in a solution containing a titanium compound such as titanium tetrachloride, and thus, a coating film is formed on the glass flakes. The precipitation of rutile-type titanium oxide on the glass flakes can be caused by adding an alkaline compound or alkaline solution to the titanium compound-containing solution having a temperature of 55 to 85°C and a pH of 1.3 or less. A tin treatment of preliminarily attaching tin or a tin compound to the glass flakes facilitates the precipitation of the rutile-type titanium oxide. By use of this method, a rutile-type titanium oxide film can be formed without the need for heating for crystal transformation. The method for forming the titanium oxide film or any other films is not limited to a liquid phase film formation method, but a gas phase film formation method represented by sputtering may also be employed.

From the optical interference film as a multilayer film, a wider variety of interference light can be obtained. Here, examples of the layered structures of optical interference films are simply listed below.
- Flaky substrate / silicon oxide / titanium oxide
- Flaky substrate / silicon oxide / iron oxide
- Flaky substrate / silicon oxide / titanium oxide / iron oxide
- Flaky substrate / silicon oxide / titanium oxide / aluminum oxide
- Flaky substrate / silicon oxide / titanium oxide / silicon oxide / titanium oxide
- Flaky substrate / titanium oxide / iron oxide
- Flaky substrate / titanium oxide / aluminum oxide
- Flaky substrate / titanium oxide / silicon oxide / titanium oxide
- Flaky substrate / tin oxide / titanium oxide
- Flaky substrate / aluminum oxide / titanium oxide
- Flaky substrate / iron oxide / titanium oxide

In the above-described layered structures, "/" indicates a boundary between the layers. Each layer may be a layer including the aforementioned oxide as the main component or a layer composed of the aforementioned oxide. Note that the optical interference film is preferably formed to cover the entire surface of the flaky substrate, but for the sake of simplicity, only the multilayer film on one of the main surfaces of the flaky substrate is described above.

### (Composite particle)

A composite particle includes a pigment particle and an oxide particle. A composite particle may include one pigment particle and a plurality of oxide particles, or may include a plurality of pigment particles and a plurality of oxide particles. A composite particle may include 5 or more, 10 or more, 30 or more, 50 or more, or in some cases, 100 or more oxide particles. The number of pigment particles that can be included in the composite particle may also be equal to or greater than the lower limits described above. The upper limits for the number of pigment particles and oxide particles both of which can be included in the composite particle are not particularly limited, but may be, for example, 10,000 or less, 5,000 or less, 3,000 or less, 1,000 or less, or even 500 or less.

### (Pigment particle)

A pigment may include an inorganic pigment and/or an organic pigment. A pigment may include an organic pigment. A pigment may be formed of a non-metallic material. Examples of the inorganic pigment include: yellow pigments such as iron oxide yellow, composite oxide yellow, and bismuth yellow; red pigments such as molybdenum red, and Bengal red (red iron oxide red); green pigments such as chromium oxide green, chromium hydroxide green, and composite oxide green; blue pigments such as dark blue (Prussian blue), azure-blue (ultramarine blue), and composite oxide blue (for example, cobalt blue); and purple pigments such as manganese violet. Examples of the organic pigment include an azo pigment, an isoindoline • isoindolinone pigment, a metal complex pigment, a phthalocyanine pigment, a quinacridone pigment, a dioxazine pigment, a diketopyrrolopyrrole pigment, a quinophthalon pigment, a perylene pigment, a thioindigo pigment, an anthraquinone pigment, and a triarylmethane pigment.

The azo pigment may be either a soluble azo pigment or an insoluble azo pigment. The soluble azo pigment is typically manufactured by performing a coupling reaction followed by insolubilization (lake formation) using salt or the like of metal such as barium, and is therefore also referred to as an azo lake pigment. Examples of the azo lake pigment include pigments based on β-naphthol, β-oxynaphthoic acid, naphthol AS, acetoacetic arylide, and naphthalene sulfonic acid. The azo lake pigments often exhibit color tones ranging from yellow to red. The azo pigments, represented by an azo lake pigment, have high coloring power and excellent vividness, making them suitable for enhancing the effects of the glitter pigment of the present embodiment.

There is no particular limitation on the size of the pigment particle, and the particle may have a size suitable for functioning as a pigment. The average particle diameter of the pigment particles is, for example, 50 nm or more and 1 µm or less. The lower limit of this average particle diameter may be 70 nm or more, 80 nm or more, 100 nm or more, or even 150 nm or more. The upper limit of this average particle diameter may be 800 nm or less, 700 nm or less, 600 nm or less, or even 500 nm or less.

### (Oxide particle)

An oxide particle may include at least one oxide selected from the group consisting of silicon oxide, aluminum oxide, zirconium oxide, titanium oxide, zinc oxide, tin oxide, niobium oxide, cerium oxide, nickel oxide, chromium oxide, vanadium oxide, and iron oxide. The oxide particle may include at least one oxide selected from the group consisting of silicon oxide, aluminum oxide, zirconium oxide, titanium oxide, and zinc oxide. The oxide particle may include silicon oxide, or may include silicon oxide as the main component. The silicon oxide has high optical transmittance and is basically colorless, making it particularly suitable for use in combination with a pigment.

There is no particular limitation on the size of the oxide particles, and the average particle diameter is, for example, 5 nm or more and 500 nm or less. The lower limit of this average particle diameter may be 10 nm or more, 30 nm or more, or even 50 nm or more. The upper limit of the average particle diameter may be 500 nm or less, 200 nm or less, or even 150 nm or less. The average particle diameter of the oxide particles may be smaller than the average particle diameter of the pigment particles.

The oxide particles prevent the plurality of pigment particles from aggregating in a direct contact state on the optical interference film. Aggregation of the pigment particles alone can increase the apparent particle diameter of the pigment particles and may cause them to detach from the optical interference film. The detachment of pigment particles inhibits exhibition of reflective color resulting from the combination of the optical interference film and the pigment particles. There is a possibility that the oxide particles impart the effect of retaining the pigment particles on the optical interference film, by means of a hydroxyl group on its surface.

Another unexpected finding is that the oxide particle itself is capable of enhancing the vividness of the color expressed by combining an optical interference film with a pigment particle. There is a possibility that since the oxide particles are interposed between the pigment particles, the oxide particles prevent a wide area of the surface of the optical interference film from being coated with only the pigment particles, thereby improving the visibility of the reflected light from the optical interference film.

### (Oxide particle / pigment particle)

The ratio of an amount of oxide particles to an amount of pigment particles is not particularly limited, but may be, for example, 0.25 or more, and 1.8 or less, in terms of mass. The lower limit of this ratio may be, for example, 0.3 or more, 0.4 or more, 0.5 or more, 0.7 or more, even 0.8 or more, or in some cases, 1.0 or more. The upper limit of this ratio may be, for example, 1.8 or less, 1.75 or less, even 1.7 or less, or in some cases, 1.6 or less. By appropriately adjusting the ratio of the oxide particles to the pigment particles, detachment of the pigment particles can be reliably suppressed, and the vividness of the color expressed by the glitter pigment may be further improved.

### (Composite particle / glitter pigment)

The ratio of the amount of composite particles to the amount of glitter pigment (the total amount of pigment) is not particularly limited, but may be, for example, 0.05% or more, and may be 70% or less, in terms of mass. The lower limit of this ratio may be, for example, 0.1% or more, 0.5% or more, or even 1% or more. The upper limit of this ratio may be, for example, 50% or less, 20% or less, or even 10% or less.

### (Attachment of composite particle)

Preferably, the composite particle is attached such that at least a portion of the surface of the optical interference film is exposed, or in other words, such that the composite particle does not coat the optical interference film, because it is suitable for enhancing the vividness of the color expressed by the glitter pigment.

### (Additional film)

The glitter pigment of the present embodiment may further include a film to coat the composite particle. This configuration is desirable for reliably suppressing detachment of the composite particle. The additional film may contain an oxide in a list of examples of oxides constituting an optical interference film. However, the additional film may affect the color expressed by the glitter pigment. As to the glitter pigment, at least the composite particle may be exposed, or in some cases, the composite particle and a portion of the surface of the optical interference film may be exposed.

### (Hue)

In the L*C*h color system, h indicates the hue angle. Here, h is in a relation of h = tan⁻¹(b^{*}/a^{*}) with a* and b* in the L*a*b* color system. In the glitter pigment of the present embodiment, the difference between h of the reflected light from the optical interference film and h of the reflected light from the pigment particle may be 100° or less, 80° or less, or even 60° or less. As to the optical interference film and the pigment used in Examples, the difference in their h values is 60° or less. The h of the reflected light from the optical interference film can be measured specifically as the h of the flaky substrate with the optical interference film. The h can be measured using the color evaluation method described in Examples. In a case where the difference is greater than 180°, the difference in their h values is indicated by the value obtained by subtracting the difference from 360°. Therefore, the difference between h₁ = 340° and h₂ = 20° is not 320°, but 40°. In the glitter pigment of the present embodiment, c* is not particularly limited but may be 30 or more, 40 or more, even 45 or more, or in some cases, 50 or more. c^{*} is an indicator of color vividness, known as chroma, and a relation c* = ((a^{*})² + (b^{*})²)^{1/2} holds.

### (Pigment-containing composition and pigment-containing painted article)

The glitter pigment according to the present embodiment can be used, for example, by blending the glitter pigment into various compositions. In another aspect, the present invention provides a pigment-containing composition containing the glitter pigment of the present invention. Examples of the pigment-containing compositions include at least one selected from paints, inks, cosmetics, and resin compositions. An example of the resin compositions is an artificial marble molded product.

In yet another aspect, the present invention provides a pigment-containing painted article including a paint film containing the glitter pigment of the present invention and a substrate material supporting the paint film. The pigment-containing painted article may be painted paper. In this case, the substrate material is paper, but the substrate material is not limited to paper and may be metal, resin, ceramics, or the like. The paint film may be formed of the pigment-containing composition of the present invention or may be formed by applying the pigment-containing composition of the present invention onto a substrate material.

Since specific examples of the pigment-containing composition and the pigment-containing painted article are well known, explanations thereof is omitted here except for the following description related to cosmetics.

Examples of cosmetics include facial cosmetics, makeup cosmetics, and hair cosmetics. In particular, the glitter pigment of the present embodiment is preferably used in makeup cosmetics such as eye shadows, nail enamels, eyeliners, mascaras, lipsticks, and face powders. The forms of cosmetics are not particularly limited, but the examples include powder, cake, pencil, stick, ointment, liquid, lotion, and cream.

The present invention will now be described in more detail with reference to Examples.

### (Color evaluation)

Spectrophotometric measurements were performed, using a spectrophotometer "CM-5" (manufactured by KONICA MINOLTA, INC.), on powders of glitter pigments obtained from Examples and Comparative Example and a powder of a substrate pigment (titanium oxide-coated glass flake) used here. The powder samples were filled into a micro petri dish (φ3 mm) and provided for color measurement. Color measurement was performed using a D65 light source by the SCE (specular component excluded) method under the condition of 2° viewing angle.

### (Detachment test)

Into a beaker, 0.1 g of the glitter pigment powder and 20 mL of water were introduced and stirred at approximately 200 rpm for 24 hours using a magnetic stirrer with a stir bar. After the stirring, the color of the supernatant was visually observed to see whether the pigment particles had been detached from the glitter pigment.

### (Amount of pigment particle attached)

The concentration of the organic substance contained in the glitter pigment was measured using a thermogravimetry differential thermal analysis (TG-DTA) device. The thus obtained value was divided by the organic component concentration (50 mass%) in the pigment that constitutes the pigment particles, so that the amount of pigment particles attached was determined.

### (Amount of oxide particle attached)

The thus obtained glitter pigment was heated and dissolved with sodium carbonate and boric acid, to which hydrochloric acid was further added to dissolve the glitter pigment. This was thoroughly washed with hydrochloric acid and hot water, and solid-liquid separation was performed using filter paper. A silica content in the filtrate was determined using a molybdenum blue absorption spectrophotometric method. A silica content in the solid was determined by the difference in weight before and after hydrofluoric acid washing. Specifically, thermal gravimetric analysis was performed on solids obtained by the solid-liquid separation and on solids obtained by dissolving and washing with hydrofluoric acid, thereby determining the silica content based on the difference in weight. The silica concentration in the glitter pigment was determined by summing the silica content in the filtrate and the silica content in the solids. Furthermore, the silica concentration in the titanium oxide-coated glass flake used as the substrate material was determined similarly, and the amount of silica (silicon oxide) constituting the oxide particle attached was calculated from the difference between the silica content in the glitter pigment and the silica content in the substrate material.

### <Preparation of glitter pigment>

### (Example 1)

Ethanol and a pigment were mixed to achieve a pigment concentration of 3 mass%, and the mixture was dispersed in a bead mill using 0.3 mm zirconia balls, whereby a pigment dispersion liquid was obtained. For the pigment, red 202 as an azo pigment was used. The average particle diameter of the obtained pigment was in a range of 100 nm to 200 nm. Meanwhile, 51.3 g of tetramethyl orthosilicate (TMOS) was weighed into a flask, and 623.7 g of pure water was added while stirring. The mixture was then stirred for another hour, whereby a TMOS hydrolysis solution having a silica-equivalent concentration of 3 mass% as obtained. Next, the pigment dispersion liquid of an amount indicated in Table 1 was added to this hydrolysis solution, whereby a TMOS hydrolysis solution containing pigment dispersed was obtained.

Separately, 891 g of pure water and 0.1 g of 1 N TMAH (tetramethyl orthosilicate) were introduced into a flask, and 100 g of titanium oxide-coated glass flake ("METASHINE (registered trademark) MT1080RR" manufactured by Nippon Sheet Glass Co., Ltd.; average particle diameter: 80 µm, thickness :1.3 µm) was further added and stirred to obtain a glass flake dispersion liquid. While stirring the glass flake dispersion liquid, the pigment-dispersed TMOS hydrolysis solution was added dropwise at a rate of 3.3 mL/min, such that the mass of the added silica (silicon oxide) reached the values shown in Table 1. During the addition of the TMOS hydrolysis solution, 1 N TMAH was also added dropwise to maintain the pH within the range of 8 to 9. The resulting dispersion liquid was then spray-dried to remove the solvent, and the resulting powder was dried at 150°C to obtain a powder of the glitter pigment in which the pigment and silicon oxide particles were made composite and attached to the titanium oxide film.

### (Examples 2-5 and Comparative Example 1)

A glitter pigment was obtained in the same manner as in Example 1, except that the amount of the pigment dispersion liquid added and the dropwise amount of the pigment-dispersed TMOS hydrolysate solution in terms of silica equivalent were modified as indicated respectively in Table 1. TMOS was not used in Comparative Example 1.

**[Table 1]**

| | Amount of pigment dispersion liquid added | Amount of TMOS added | Amount of pigment attached | Amount of oxide attached | Oxide amount / pigment amount | L^{*} | a* | b* | c* | Detachment |
|---|---|---|---|---|---|---|---|---|---|---|
| Comparative Example 1 | 82.0 | 0.00 | 0.35 | 0.00 | 0.00 | 72.5 | 29.7 | -2.1 | 29.8 | Yes |
| Example 1 | 82.7 | 1.03 | 0.94 | 0.51 | 0.54 | 58.3 | 48.2 | 4.0 | 48.4 | No |
| Example 2 | 83.3 | 1.76 | 0.97 | 1.12 | 1.16 | 52.2 | 53.4 | 11.1 | 54.5 | No |
| Example 3 | 84.3 | 2.96 | 0.98 | 1.33 | 1.35 | 52.7 | 52.5 | 9.7 | 53.4 | No |
| Example 4 | 85.3 | 4.00 | 0.95 | 1.43 | 1.51 | 58.1 | 51.7 | 1.0 | 51.7 | No |
| Example 5 | 53.3 | 1.08 | 0.92 | 0.61 | 0.66 | 59.5 | 48.9 | 2.25 | 49.0 | No |

| | | | | | | | | | | |
|---|---|---|---|---|---|---|---|---|---|---|
| • Amount added and amount attached each are amounts (g) relative to 100 g of flaky substrate with optical interference film • Amount of TMOS added is expressed as silica equivalent | | | | | | | | | | |

"Oxide amount / pigment amount", which is a ratio of the amount of oxide to the amount of pigment, both being calculated from the respective raw materials added, are as follows: 0 in Comparative Example 1, 0.42 in Example 1, 0.70 in Example 2, 1.17 in Example 3, 1.56 in Example 4, and 0.68 in Example 5.

An observation using SEM revealed that a composite particle including a pigment particle and an oxide particle was attached to the surface of the optical interference film in each Example, though a portion of the surface of the optical interference film was exposed.

### <Preparation of pigment-containing composition>

### (Eye shadow)

An extender pigment and a spherical powder of Category A shown in Table 2 were placed in a disposable cup and mixed with a spatula. The mixture was then introduced into a mixer and mixed for 1 minute. Next, a color pigment and a glitter pigment were added, and the mixture was mixed in the mixer for further 30 seconds. Subsequently, raw materials of Category B were introduced into the mixer, and the mixture was mixed for further 1 minute. The resulting powder was collected, an appropriate amount thereof was placed in a mold and pressed to obtain an eye shadow.

**[Table 2]**

| (mass part) | | | | |
|---|---|---|---|---|
| Category | Classification | Product name (raw material) | Manufacturer | Formulation |
| A | Extender pigment | JA-46R (talc) | Asada Milling Co., Ltd. | 8.5 |
| | | GMS-4C (sericite) | KINSEI MATEC CO.,LTD. | 1.6 |
| | | NK-10G (synthetic mica) | Nihon Koken Kogyo Co., Ltd. | 1.6 |
| | Color pigment | IRS30S03X (30% red 202-encapsulated silica spherical particle, 8 µm) | Nippon Sheet Glass Co., Ltd. | 0.2 |
| | Spherical powder | IWS22S13 (30% titanium oxide-encapsulated silica spherical particle, 4 µm) | Nippon Sheet Glass Co., Ltd. | 0.8 |
| | Glitter pigment | Example 2 | | 5.2 |
| B | Oil component | NOMUCOAT (a-olefin oligomer) | Nisshin OilliO Group, Ltd. | 0.20 |
| | | P-150 (petrolatum) | NIKKO RICA CORPORATION | 1.20 |
| | | KF-96-6 (silicone oil) | Shin-Etsu Chemical Co., Ltd. | 0.40 |
| | Surfactant | COSMOL 182V (sorbitan sesquistearate) | Nisshin OilliO Group, Ltd. | 0.20 |
| | Antioxidant | E-Mix D | Eisai Food & Chemical Co., Ltd | 0.01 |
| | Preservative | Ethyl paraben | Tokyo Chemical Industry | 0.07 |
| Total | | | | 20 |

A vivid red color was observed in the obtained eye shadow. When the eye shadow was applied onto the back of the hand and observed, a transparent red color without any dullness was observed from any angle. In particular, a lustrous red color was observed in the specular direction.

### (Lip gloss)

The raw materials of Category A shown in Table 3 were weighed into a disposable cup, heated in a water bath at a temperature in a range of 80 to 90°C, and mixed while melting the raw materials. Further, the glitter pigment of Category B was added and mixed, then the mixture was poured into a transparent container.

**[Table 3]**

| (mass part) | | | | |
|---|---|---|---|---|
| Category | Product name (raw material) | Manufacturer | Formulation 1 | Formulation 2 |
| A | SALACOS 5408 (pentaerythrityl tetraethylhexanoate) | Nisshin OilliO Group Ltd. | 46.7 | 46.7 |
| | COSMOL 168ARV (dipentaerythrityl hexahydroxystearate/ hexastearate/hexarosinate) | Nisshin OilliO Group, Ltd. | 4.0 | 4.0 |
| | COSMOL 222 (diisostearyl malate) | Nisshin OilliO Group, Ltd. | 37.0 | 37.0 |
| | NOMUCOAT TAB (octyl methoxycinnamate) | Nisshin OilliO Group, Ltd. | 2.7 | 2.7 |
| | PARSOL 1789 (t-butyl methoxydibenzoylmethane) | DSM Nutrition Japan | 0.1 | 0.1 |
| | AEROSIL R 972 (fumed silica, surface-treated with dimethyl dichlorosilane) | NIPPON AEROSIL CO., LTD. | 0.7 | 0.7 |
| | Rheopearl (polymeric ester) | Chiba Flour Milling Co., Ltd. | 7.7 | 7.7 |
| | Ethyl paraben | | 0.2 | 0.2 |
| B | Glitter pigment (Example 2) | | 1.0 | |
| | Glitter pigment (Comparative Example 1) | | | 1.0 |
| Total | | | 100.1 | 100.1 |

The lip gloss of Formulation 1 had a striking pearlescent appearance of vivid pink color. The lip gloss of Formulation 2 exhibited excellent transparency and brightness, but the red pigment was found detached and aggregating in observation. The lip gloss of Formulation 1, which was applied to the lips, imparted a glossiness and a healthy flush of color. In the meantime, the lip gloss of Formulation 2 imparted a glossiness but failed to impart a healthy flush of color.

### (Embodiments of the present invention)

As described above, the present invention provides, as a first aspect, a glitter pigment including:
a flaky substrate;
an optical interference film on the flaky substrate; and
a composite particle including a pigment particle and an oxide particle, the composite particle being attached to the optical interference film.

The present invention provides, as a second aspect, the glitter pigment according to the first aspect, wherein
the pigment particle includes an organic pigment.

The present invention provides, as a third aspect, the glitter pigment according to the first or the second aspect, wherein
the oxide particle includes at least one selected from the group consisting of silicon oxide, aluminum oxide, zirconium oxide, titanium oxide, zinc oxide, iron oxide, tin oxide, niobium oxide, and cerium oxide.

The present invention provides, as a fourth aspect, the glitter pigment according to the third aspect, wherein
the oxide particle includes silicon oxide.

The present invention provides, as a fifth aspect, the glitter pigment according to any one of the first to the fourth aspects, wherein
the optical interference film includes titanium oxide.

The present invention provides, as a sixth aspect, the glitter pigment according to any one of the first to the fifth aspects, wherein
the flaky substrate is a glass flake.

The present invention provides, as a seventh aspect, the glitter pigment according to any one of the first to the sixth aspects, wherein
a ratio of an amount of the oxide particle to an amount of the pigment particle is 0.25 or more in terms of mass.

The present invention provides as an eighth aspect, the glitter pigment according to any one of the first to the seventh aspects, wherein
a ratio of an amount of the oxide particle to an amount of the pigment particle is 1.8 or less in terms of mass.

The present invention provides, as a ninth aspect, a pigment-containing composition including the glitter pigment according to any one of the first to the eighth aspects.

The present invention provides, as a tenth aspect, a pigment-containing painted article including:
a paint film including the glitter pigment according to any one of the first to the eighth aspects; and
a substrate material supporting the paint film.

## Claims

1. A glitter pigment comprising:
a flaky substrate;
an optical interference film on the flaky substrate; and
a composite particle comprising a pigment particle and an oxide particle, the composite particle being attached to the optical interference film.

2. The glitter pigment according to claim 1, wherein
the pigment particle comprises an organic pigment.

3. The glitter pigment according to claim 1, wherein
the oxide particle comprises at least one selected from the group consisting of silicon oxide, aluminum oxide, zirconium oxide, titanium oxide, zinc oxide, iron oxide, tin oxide, niobium oxide, and cerium oxide.

4. The glitter pigment according to claim 3, wherein
the oxide particle comprises silicon oxide.

5. The glitter pigment according to claim 1, wherein
the optical interference film comprises titanium oxide.

6. The glitter pigment according to claim 1, wherein
the flaky substrate is a glass flake.

7. The glitter pigment according to claim 1, wherein
a ratio of an amount of the oxide particle to an amount of the pigment particle is 0.25 or more in terms of mass.

8. The glitter pigment according to claim 1, wherein
a ratio of an amount of the oxide particle to an amount of the pigment particle is 1.8 or less in terms of mass.

9. A pigment-containing composition comprising the glitter pigment according to any one of claims 1 to 8.

10. A pigment-containing painted article comprising:
a paint film comprising the glitter pigment according to any one of claims 1 to 8; and
a substrate material supporting the paint film.
